(19) Europäisches Patentamt
European Patent Office
Office européen des brevets

(11) Veröffentlichungsnummer: 0 355 565 A1

(12) # EUROPÄISCHE PATENTANMELDUNG

(21) Anmeldenummer: 89114616.9

(22) Anmeldetag: 08.08.89

(51) Int. Cl.4: C07D 405/04 , C07D 405/14 , A61K 31/44 , A61K 31/40

Patentansprüche für folgende Vertragsstaaten: ES + GR.

(30) Priorität: 13.08.88 DE 3827532

(43) Veröffentlichungstag der Anmeldung: 28.02.90 Patentblatt 90/09

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Englert, Heinrich Christian, Dr.
Stormstrasse 13
D-6238 Hofheim am Taunus(DE)
Erfinder: Mania, Dieter, Dr.
Goethestrasse 43
D-6240 Königstein/Taunus(DE)
Erfinder: Utz, Roland, Dr.
Sudetenstrasse 1862
D-6101 Messel(DE)
Erfinder: Schölkens, Bernward, Dr.
Hölderlinstrasse 62
D-6233 Kelkheim(Taunus)(DE)

(54) **6-Aroyl-substituierte 3,4-Dihydro-2H-benzopyrane, Verfahren zu ihrer Herstellung, ihre Verwendung sowie pharmazeutische Präparate auf Basis dieser Verbindungen.**

(57) Verbindung I

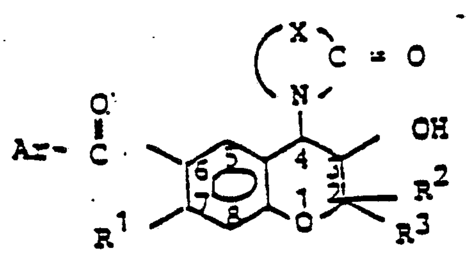

mit
$R^1$ gleich H, Alkoxy, Alkyl, $NR^4R^5$ und
$R^2$,$R^3$ gleich H, Alkyl oder gemeinsam eine Kette,
Ar ein gegebenenfalls substituierter Aromat,
X eine $(CH_2)$-Kette
haben hervorragende Wirksamkeit als Antihypertensiva, als Coronartherapeutika, als Mittel zur Behandlung der Herzinsuffizienz, der cerebralen bzw. peripheren Durchblutungsstörungen oder von Darmmotilitätsstörungen, vorzeitiger Wehentätigkeit, Obstruktionen der Atemwege oder der ableitenden harnwege oder der Gallenwege oder als Spasmolytika.

EP 0 355 565 A1

## 6-Aroyl-substituierte 3,4-Dihydro-2H-benzopyrane, Verfahren zu ihrer Herstellung, ihre Verwendung sowie pharmazeutische Präparate auf Basis dieser Verbindungen

Die Erfindung betrifft 3,4-Dihydro-2H-benzo[b]pyrane der Formel I

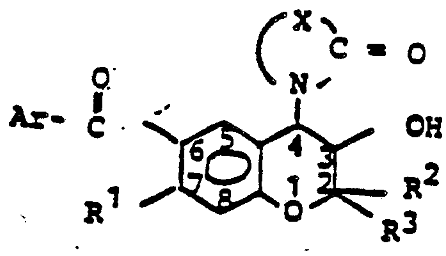

I

in welcher
$R^1$ für H, OH, $(C_1\text{-}C_2)$-Alkoxy, $(C_1\text{-}C_2)$-Alkyl oder $NR^4R^5$ steht, wobei $R^4$ und $R^5$ gleich oder verschieden sind und für H, $(C_1\text{-}C_2)$-Alkyl oder $(C_1\text{-}C_3)$-Alkylcarbonyl stehen,
$R^2$,$R^3$ gleich oder verschieden sind und für H oder Alkyl mit 1-4 C-Atomen stehen,
$R^2$,$R^3$ zusammen eine Kette $(CH_2)_n$ bilden mit n = 3,4,5 oder 6
Ar für ein aromatisches oder heteroaramatisches System steht, das unsubstituiert oder durch 1 bis 3 gleiche oder verschiedene Reste $(C_1\text{-}C_2)$-Alkyl, $(C_1\text{-}C_2)$-Alkoxy, Halogen, Trifluormethyl, CN, $NO_2$, CO-$(C_1\text{-}C_2)$-Alkyl $SO_m$-$(C_1\text{-}C_2)$-Alkyl mit m = 1 oder 2 substituiert ist
X für eine Kette $(CH_2)_r$ steht, die durch einen Heteroatom 0, S oder $NR^6$ unterbrochen sein kann, wobei $R^6$ H oder $(C_1\text{-}C_4)$-Alkyl bedeutet und r für die Zahlen 2, 3, 4 oder 5 steht
oder
X für eine Kette $(CH)_{n'}$ steht mit n′ = 4 oder 6
oder
X für eine Kette $-CH_2-(CH=CH)_{n''}-$ steht mit n″ = 1 oder 2,
X unsubstituiert oder substituiert ist durch einen Substituenten A mit A in der Bedeutung $(C_1\text{-}C_2)$-Alkyl, $(C_1\text{-}C_2)$-Alkoxy, COOH, $NO_2$, $NH_2$, Halogen

Unter einem aromatischen System Ar wird vorzugsweise Phenyl, Naphthyl oder Biphenylyl verstanden, ein 5- oder 6-gliedriges heteroaromatisches System Ar ist vorzugsweise ein Rest eines 5- oder 6-gliedrigen O, N und/oder S-heterocyclischen Ringes, insbesondere Furyl, Thienyl, Isothiazolyl, Oxazolyl, Isoxazolyl, Pyrazolyl, Imidazolyl, Thiazolyl, Pyridyl, Pyrazinyl, Pyrimidinyl, Pyridazinyl, Triazinyl.

Unter Halogen wird F, Cl, Br oder J, vorzugsweise F und Cl verstanden.

Die C-Atome 3 und 4 des 3,4-Dihydro-2H-benzo[b]pyransystems (nachfolgend auch kurz "Chromansystem" genannt) der Formel I sind asymmetrisch substituiert. Die Erfindung betrifft dabei nur solche Verbindungen, die an diesen Zentren entgegengesetzte Konfigurationen, also eine "trans-Orientierung" der Substituenten an diesen C-Atomen aufweisen. Enthält einer der Substituenten $R^1$, $R^2$ und/oder $R^3$ Asymmetriezentren oder, falls $R^2$ und $R^3$ ungleich sind (und somit ein asymmetrisches Kohlenstoffatom erzeugen), gehören Verbindungen mit sowohl S- als auch R-konfigurierten Zentren zur Erfindung.

Die Verbindungen können als optische Isomere, als Diasteroisomere, als Racemate oder als Gemische derselben vorliegen.

Bevorzugt sind Verbindungen I bei den $R^1$ bis $R^3$ und Ar die oben genannten Bedeutungen haben und X für eine Kette $(CH_2)_r$ steht, die unsubstituiert oder durch A in der oben genannten Bedeutung substituiert ist.

Bevorzugt sind Verbindungen I bei denen $R^1$ bis $R^3$ und Ar die oben genannte Bedeutung haben, X jedoch für eine Kette $(CH)_{n'}$ steht mit n′ = 4.

Bevorzugt sind Verbindungen I bei denen $R^1$ bis $R^3$ und Ar die oben genannten Bedeutungen haben, X jedoch für eine Kette $CH_2-(CH=CH)_{n''}-$ steht mit n″ = 1.

Bevorzugt sind ferner Verbindungen I mit $R^1$ bis $R^3$ und X in den oben genannten Definitionen, bei denen Ar für Phenyl steht, das unsubstituiert oder einfach durch Halogen, $(C_1\text{-}C_2)$-Alkyl, $(C_1\text{-}C_2)$-Alkoxy substituiert ist.

Ganz besonders bevorzugt sind Verbindungen, bei denen X für eine Kette $(CH_2)_r$ mit r = 3 oder 4 und Ar für Phenyl steht, das unsubstituiert oder einfach durch Halogen, $(C_1\text{-}C_2)$-Alkyl, $(C_1\text{-}C_2)$-Alkoxy substituiert ist, sowie mit $R^1$-$R^3$ in den eingangs erwähnten Definitionen.

Eine weitere bevorzugte Untergruppe sind solche Verbindungen I bei denen $R^2$, $R^3$, Ar und X ein eingangs erwähnten Bedeutungen haben, $R^1$ jedoch für H steht.

Hiervon sind besonders bevorzugt solche Verbindungen bei denen $R_2$,$R_3$ jeweils für ($C_1$-$C_2$)-Alkyl steht, X für $(CH_2)_r$ mit r=3 oder 4, Ar für Phenyl steht, das unsubstituiert oder einfach durch Halogen, ($C_1$-$C_2$)-Alkyl, ($C_1$-$C_2$)-Alkoxy substituiert ist.

Ähnliche Verbindungen sind aus der EP-Offenlegungsschriften 0277612 und 0277611 bekannt. Sie enthalten jedoch eine $SO_n$-Brücke zwischen dem Chromansystem und dem Substituenten Ar.

Mit den Verbindungen I wurde nun eine neue Substanzklasse mit wertvollen pharmakologischen Eigenschaften gefunden. Tierexperimentelle Untersuchungen zeigten, daß sie sich für die Behandlung des gestörten cardiovaskulären Systems eignen, beispielsweise für die Behandlung der Hypertonie, der Herzinsuffizienz oder von Durchblutungsstörungen des Coronarsystems wie etwa Angina. Cerebrale und periphere Durchblutungsstörungen werden ebenfalls günstig beeinflußt. Darüber hinaus können Verbindungen I glattmuskuläre Organe wie Uterus, Bronchien, Darm und Gallengänge, die ableitenden Harnwege (Ureter, Harnblase und Urethra) im Sinne einer Spasmolyse beeinflussen. Sie eignen sich deshalb auch zur Behandlung von Krankheiten, die mit Spasmen dieser Organe verbunden sind, beispielsweise zur Behandlung von vorzeitiger Wehentätigkeit bei der Schwangerschaft, von Koliken der Harnleiter oder der Galle, von obstruktiven Atemwegskrankheiten wie Asthma, von Störungen der Darmmotilität wie etwa des irritablen Colons oder der Inkontinenz der Harnblase.

Die Erfindung betrifft weiterhin ein Verfahren zur Herstellung der Verbindungen I, dadurch gekennzeichnet, daß man

a) Verbindungen der Formel II

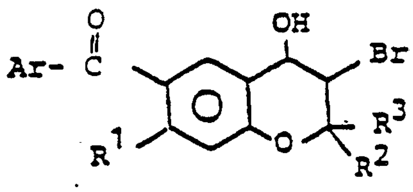

II,

in denen $R^1$ bis $R^3$ und Ar wie oben definiert sind
umsetzt mit Lactamen der Formel III

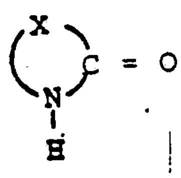

III,

b) Verbindungen der Formel IV

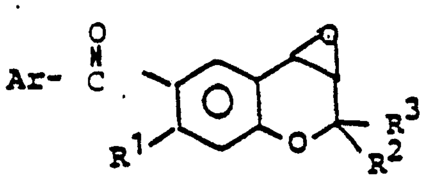

IV,

in denen $R^1$ bis $R^3$ und Ar wie oben definiert sind,
umsetzt mit den Lactamen der Formel III

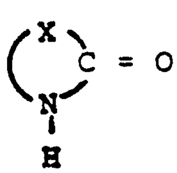

c) Verbindungen der Formel V

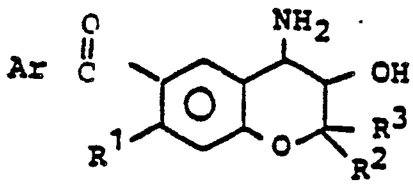 V,

in denen $R^1$ bis $R^3$ und Ar wie oben definiert sind,
acyliert zu den Verbindungen VI

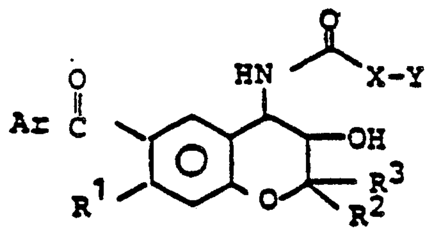 VI,

in denen Y eine Fluchtgruppe, wie etwa Chlor oder Brom, ist und $R^1$ bis $R^3$ und Ar wie oben definiert sind, und diese zu den Verbindungen I cyclisiert

d) Verbindungen der Formel VII

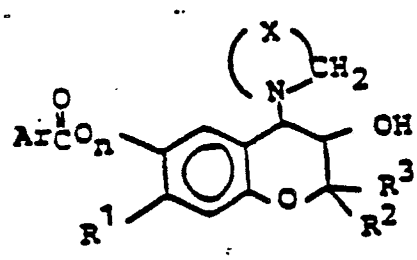 VII,

in denen $R^1$ bis $R^3$ und Ar wie oben definiert sind,
zu den Verbindungen I oxidiert.

Werden die Verbindungen I nach den Methoden a) oder b) hergestellt, so geschieht dies dadurch, daß man die Verbindungen II oder IV in einem geeigneten Lösemittel, vorzugsweise in dipolar aprotischen Lösemitteln wie etwa Dimethylsulfoxid umsetzt mit den Lactamen III, vorzugsweise unter Einwirkung von Basen, wie etwa Natriumhydrid, K-tert.butylat oder ähnlichen, für Lactam-N-Alkylierungen bekanntermaßen geeigenten Basen. Die Reaktionstemperatur ist dabei im weiten Bereich variierbar; vorzugsweise wird zwischen 0° und Zimmertemperatur gearbeitet oder bei Temperaturen, die leicht oberhalb der Zimmertemperatur liegen können.

Lactame der Formel III sind in vielen Fällen bekannt, oder können leicht nach literaturbekannten Methoden hergestellt werden. Verbindungen II oder IV sind neu. Sie können beispielsweise nach folgendem Syntheseweg hergestellt werden:

Verbindungen der Formel VIII

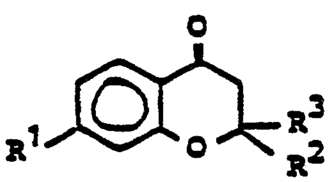 VIII,

in denen $R^1$, $R^2$ und $R^3$ wie oben definiert sind, werden mit Säurechloriden ArCOCl in an sich bekannter

Weise nach Art der Freidel-Crafts Acylierung umgesetzt zu Verbindungen der Formel IX

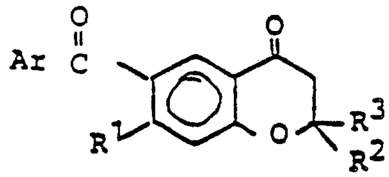

IX,

in denen $R^1$, $R^2$, $R^3$ und Ar und n wie oben definiert sind. Diese werden durch Reduktionen unter Standardbedingungen etwa durch $NaBH_4$ in Methanol umgesetzt zu den Verbindungen X

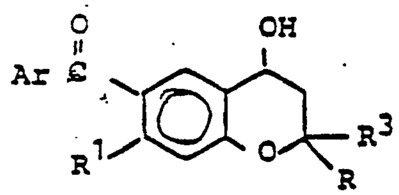

X

und anschließend einer Wasserabspaltung, etwa durch Pyridin/Phosphoroxychlorid, unterworfen, wobei Verbindungen der Formel XI entstehen:

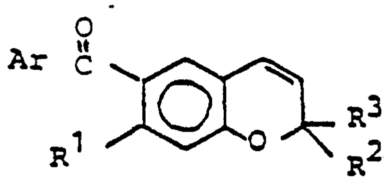

XI .

Verbindungen XI lassen sich nun leicht nach Standardmethoden in die Epoxide IV oder die Bromhydrine II umwandeln.

Steht bei dieser Reaktionssequenz $R^1$ in der Bedeutung von $NH_2$ oder OH, so sind gegebenenfalls Schutzgruppen wie etwa die Dimethylaminomethylengruppe für $NH_2$ oder die Acetyl- oder Methylgruppe für die OH-Gruppe notwendig. Diese werden auf geeigneten Stufen, vorzugsweise nach Durchführung die in Verfahren a) oder b) beschriebenen Umsetzungen durch gängige Methoden wieder abgespalten.

Chromene der Formel XI werden in einigen Fällen durch in an sich bekannter Weise durch thermisch induzierte Cyclisierung der entsprechenden Propargylether XII

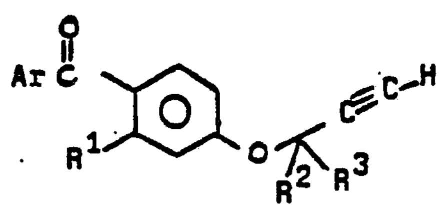

XII

hergestellt. Diese wiederum lassen sich in an sich bekannter Weise aus den Phenolen XIII und den Propargylchloriden XIV herstellen.

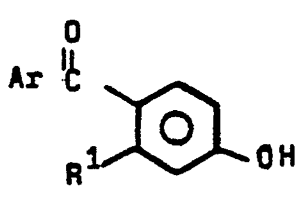

XIII

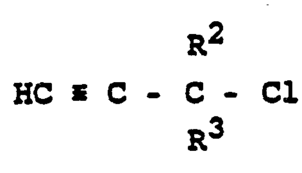

XIV

Die Verfahren c) und d) können dann besonders günstig angewandt werden, wenn enantiomerenreine Endprodukte I erwünscht sind. Verbindungen V und VII sind in Gegensatz zu Verbindungen I basisch und damit zur Salzbildung mit organischen Säuren befähigt. Durch Kristallisation mit einer geeigneten optisch einheitlichen Säure wie etwa (+)-Mandelsäure oder (+)-Milchsäure können sie in an sich bekannter Weise enantiomerein erhalten werden und in enantiomerenreine Endprodukte I nach den Verfahren c) und d) umgewandelt werden.

Enantiomerenreine Endprodukte I können aber auch aus racemischen Endprodukten I durch gängige Racematspaltungsmethoden wie etwa die chromatographische Trennung unter Verwendung von chiralen Phasen oder die Derivatisierung der racemischen Produkte mit optisch einheitlichen Säurederivaten (Esterbildung über die 3-Hydroxygruppe des Chromansystems) oder mit optisch einheitlichen Isocyanaten (Carbamatbildung über die 3-Hydroxygruppe) können angewandt werden. Die dabei erhaltenen diastereoisomeren Isocyanate oder Ester lassen sich durch gängige Methoden (Kristallisation oder Chromatographie) trennen und unter Abspaltung der optisch aktiven Hilfsgruppe an der 3-OH-Funktion in die optisch einheitlichen Endverbindungen I umwandeln. Als besonders vorteilhaft hat sich hierbei die Trennung der diastereomeren 3-Menthoxyacetate erwiesen.

Die erfindungsgemäßen Verbindungen I können wie bereits erwähnt als Antihypertensiva, als Coronartherapeutika, als Mittel zur Behandlung der Herzinsuffizienz, der cerebralen bzw. peripheren Durchblutungsstörungen oder von Darmmotilitätsstörungen, vorzeitiger Wehentätigkeit Obstruktionen der Atemwege oder der ableitenden Harnwege oder der Gallenwege oder als Spasmolytika eingesetzt werden.

Arzneimittel, die die Verbindung I enthalten, können dabei oral, parenteral, intravenös, rektal oder durch Inhalation appliziert werden, wobei die bevorzugt Applikationsform von der zu behandelnden Krankheit abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen wie Trägerstoffen, Stabilisatoren oder inerten Verdünnungsmittel vermischt und durch die üblichen Methoden in geeignete Darreichungsformen gebracht, wie Tabletten, Dragees, Steckkapseln, wäßrige, alkoholisch oder ölige Suspensionen oder wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedener genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffs der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelden Krankheit sowie von Geschlecht, Alter, Gewicht und individueller Ansprechbarkeit des zu behandelndes Säugers. Im Durchschnitt beträgt die empfohlene tägliche Dosis einer Verbindung der Formel I bei einem etwa 75 kg schweren Patienten mindestens 0,1 mg, vorzugsweise mindestens 1 g, bis maximal 100 mg, vorzugsweise bis maximal 10 mg. Bei akuten Ausbrüchen der Krankheit, etwa bei Asthmaanfällen oder bei Nierenkoliken, können dabei mehrere, z. B. bis zu 4 Einzeldosen pro Tag, erforderlich sein, während zur Prophylaxe auch eine Dosierung ausreichen kann.

Erfindungsgemäß können beispielsweise die in der folgenden Tabelle zusammengestellten Verbindungen der Formel I erhalten werden.
2,2-Dimethyl-3,4-dihydro-6-p-chlorbenzoyl-trans-4-(2-oxo-1-pyrrolidinyl)-2H-benzopyran-3-ol
2,2-Dimethyl-3,4-dihydro-6-(3-furylcarbonyl)-trans-4-(2-oxo-1-pyrrolidinyl)-2H-benzopyran-3-ol
2,2 -Dimethyl-3,4-dihydro-6-nicotinoyl-trans-4-(2-oxo-1-pyrrolidinyl)-2H-benzopyran-3-ol
2,2-Dimethyl-3,4-dihydro-6-(2-naphthylcarbonyl)-trans-4-(2-oxo-1-pyrrolidinyl)-2H-benzopyran-3-ol
2,2-Dimethyl-3,4-dihydro-6-(1-naphthylcarboxyl)-trans-4-(2-oxo-1-pyrrolidinyl)-2H-benzopyran-3-ol
2,2-Dimethyl-3,4-dihydro-6-benzoyl-7-methyl-trans-4-(2-oxo-1-pyrrolidinyl)-2H-benzopyran-3-ol
2,2-Dimethyl-3,4-dihydro-6-(2-pyrazinoyl)-trans-4-(2-oxo-1-pyrrolidinyl)-2H-benzopyran-3-ol

**Beispiel 1**

6-Benzoyl-3,4-dihydro-2,2-dimethyl-trans-4(2-oxo-1-pyrrolidinyl)-2H-benzo[b]pyran-3-ol

5,1 g (0,014 Mol) 6-Benzoyl-3-brom-3,4-dihydro-2,2-dimethyl-2H-benzo[b]pyran-4-ol werden in 85 ml Dimethylsulfoxid gelöst und mit 12,8 ml Pyrrolidin-2-on sowie mit 2,6 g NaH (90 % in Öl) versetzt und 5 h bei Zimmertemperatur gerührt. Das Reaktionsgemisch wird anschließend auf Eis/Wasser gegossen und der ausgefallene Niederschlag abgesaugt.
Weiße Kristalle von Schmp.: 209 - 211 °C

**Beispiel 2**

6-Benzoyl-3,4-dihydro-2,2-dimethyl-trans-4-(2-oxo-1-piperidinyl)-2H-benzo[b]pyran-3-ol

Analog Beispiel 1 mit Piperidin-2-on an Stelle von Pyrrolidin-2-on.
Weiße Kristalle von Schmp.: 201 - 203 °C

**Beispiel 3**

6-Benzoyl-3,4-dihydro-2,2-dimethyl-trans-4-[5′-methyl-2′-oxo-1′-pyrrolidinyl)-2H-benzo[b]pyran-3-ol

Diastereomer A und Diastereomer B

Diastereomer A:

Zu einer Lösung von 20,2 g (0,056 Mol) 6-Benzoyl-3-brom-3,4-dihydro-2,2-dimethyl-2H-benzo[b]pyran-4-ol in 100 ml Dimethylsulfoxid werden 1,8 g (0,06 Mol) 80 %iges NaH zugefügt. Nach einstündigem Rühren bei 24 °C werden weitere 2,4 g (0,08 Mol) 80 %iges NaH und 8,6 g (0,087 Mol) racemisches 5-Methyl-2-pyrrolidinon eingetragen. Nach 6 stündigem Rühren bei Zimmertemperatur wird das Reaktionsgemisch in Eis/Wasser gegossen und mit Methylenchlorid extrahiert. Die Methylenchloridphase wird mehrfach mit Wasser gewaschen, getrocknet und eingedampft und aus Essigester/Petrolether (4:1) umkristallisiert. Das Diastereomer A kristallisiert fast sauber aus.
Kristalle vom Schmp.: 221-222 °C (aus Methanol/ mit wenig DMF)
NMR ($d_6$-DMSO)
$CH_3$-Signale: $\delta$
0,83 (3 H, d)
1,20 (3 H, s)
1,50 (3 H, s)

Diastereomer B:

Die Mutterlange (Essigester/Petrolether 4:1), in der das Diastereomer B stark angereichert ist, wird eingedampft und die Verbindung über eine Kieselgelsäule mit Essigester/Petrolether (4:1) isoliert.
Kristalle vom Schmp: 196-197 °C (aus Ethanol)
NMR ($d_6$-DMSO)
$CH_3$-Signale: $\delta$
1,19 (3 H, d)
1,20 (3 H, s)
1,44 (3 H, s)

**Herstellung des Ausgangsmaterials:**

6-Benzoyl-3-brom-3,4-dihydro-2,2-dimethyl-2H-benzo[b]pyran-4-ol wurde aus 6-Benzoyl-2,2-dimethyl-2H-benzo[b]pyran mit N-Bromsuccinimid und Wasser in Dimethylsulfoxid erhalten.
Schmp.: 137 - 139 °C.

6-Benzoyl-2,2-dimethyl-2H-benzo[b]pyran wurde aus 4-Benzoylphenyl-1′,1′-dimethylpropargylether durch Refluxieren in 1,2-Dichlorbenzol erhalten.

4-Benzoylphenyl-1′,1′-dimethylpropargylether wird, wie in den europäischen Offenlegungschriften 0277612 und EP-OS 0277611 beschrieben aus 4-Hydroxybenzophenon und 1,1-Dimethylpropargylchlorid erhalten.

**Beispiel 4**

6-(2-Trifluormethylbenzoyl)-3,4-dihydro-2,2-dimethyl-trans-4-(2-oxo-1-pyrrolidinyl)-2H-benzo[b]pyran-3-ol

Analog Beispiel 1 mit 6-(2-Trifluormethylbenzoyl-3-brom-3,4-dihydro-2,2-dimethyl-2H-benzo[b]pyran-4-ol und Pyrrolidin-2-on als Ausgangsmaterial.
Weiße Kristalle vom Schmp.: 175 - 177 °C

**Beispiel 5**

6-(2-Chlorbenzoyl)-3,4-dihydro-2,2-dimethyl-trans-4-(2-oxo-1-pyrrolidinyl)-2H-benzo[b]pyran-3-ol

Analog Beispiel 1 mit 6-(2-Chlorbenzoyl)-3-brom-3,4-dihydro-2,2-dimethyl-2H-benzo[b]pyran-4-ol und Pyrrolidin-2-on als Ausgangsmaterial.
Weiße Kristalle vom Schmp.: 233 °C

**Ansprüche**

1. 3,4-Dihydro-2H-benzo[b]pyrane der Formel I

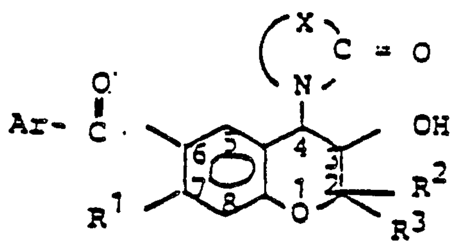

I

in welcher
$R^1$ für H, OH, $(C_1-C_2)$-Alkoxy, $(C_1-C_2)$-Alkyl oder $NR^4R^5$ steht, wobei $R^4$ und $R^5$ gleich oder verschieden sind und für H, $(C_1-C_2)$-Alkyl oder $(C_1-C_3)$-Alkylcarbonyl stehen,

$R^2$,$R^3$ gleich oder verschieden sind und für H oder Alkyl mit 1-4 C-Atomen stehen,
$R^2$,$R^3$ zusammen eine Kette $(CH_2)_n$ bilden mit n = 3,4,5 oder 6
Ar für ein aromatisches oder heteroaromatisches System steht, das unsubstituiert oder durch 1 bis 3 gleiche oder verschiedenen Reste $(C_1\text{-}C_2)$-Alkyl, $(C_1\text{-}C_2)$-Alkoxy, Halogen, Trifluormethyl, CN, $NO_2$, CO-$(C_1\text{-}C_2)$-Alkyl $SO_m$-$(C_1\text{-}C_2)$-Alkyl mit m = 1 oder 2 substituiert ist
X für eine Kette $(CH_2)_r$ steht, die durch ein Heteroatom 0, S oder $NR^6$ unterbrochen sein kann, wobei $R^6$ H oder $(C_1\text{-}C_4)$-Alkyl bedeutet und r für die Zahlen 2, 3, 4 oder 5 steht
oder
X für eine Kette $(CH)_{n'}$ steht mit n′ = 4 oder 6
oder
X für eine Kette $-CH_2-(CH=CH)_{n''}-$ steht mit n″ = 1 oder 2,
X unsubstituiert oder substituiert ist durch einen Substituenten A mit A in der Bedeutung $(C_1\text{-}C_2)$-Alkyl, $(C_1\text{-}C_2)$-Alkoxy, COOH, $NO_2$, $NH_2$, Halogen

2. Verbindung I nach Anspruch 1, dadurch gekennzeichnet, daß mindestens einer der Substituenten oder Indices folgende Bedeutung hat:
$R^1$,$R^2$,$R^3$ und Ar wie in Anspruch 1 definiert
X $(CH_2)_r$ mit r = 2,3,4 oder 5, welche Kette unsubstituiert oder wie in Anspruch 1 definiert substituiert ist.

3. Verbindung I nach Anspruch 1, dadurch gekennzeichnet, daß mindestens einer der Substituenten oder Indices folgende Bedeutung hat:
$R^1$,$R^2$,$R^3$ und Ar wie in Anspruch 1 definiert
X $(CH)_{n'}$ mit n′ gleich 4.

4. Verbindung I nach Anspruch 1, dadurch gekennzeichnet, daß mindestens einer der Substituenten oder Indices folgende Bedeutung hat:
$R^1$,$R^2$,$R^3$,X wie in Anspruch 1 definiert
Ar Phenyl, das unsubstituiert ist oder einfach substituiert durch Halogen, $(C_1\text{-}C_2)$-Alkyl, $(C_1\text{-}C_2)$-Alkoxy.

5. Verbindung I nach Anspruch 1, dadurch gekennzeichnet, daß minestens einer der Substituenten oder Indices folgende Bedeutung hat:
$R^1$,$R^2$,$R^3$ wie in Anspruch 1 definiert
X $(CH_2)_r$ mit r = 3 oder 4
Ar Phenyl, unsubstituiert oder einfach durch Halogen, $(C_1\text{-}C_2)$-Alkyl, $(C_1\text{-}C_2)$-Alkoxy substituiert.

6. Verbindung I nach Anspruch 1, dadurch gekennzeichnet, daß mindestens einer der Substituenten oder Indices folgende Bedeutung hat:
$R^1$ H
$R^2$,$R^3$, Ar und X wie Anspruch 1 definiert.

7. Verbindung I nach Anspruch 6, dadurch gekennzeichnet, daß mindestens einer der Substituenten oder Indices folgende Bedeutung hat:
$R^1$ H
$R^2$ und $R^3$ $(C_1\text{-}C_2)$-Alkyl,
X $(CH_2)_r$ mit r = 3 oder 4
Ar Phenyl, unsubstituiert oder einfach durch Halogen, $(C_1\text{-}C_2)$-Alkyl, $(C_1\text{-}C_2)$-Alkoxy substituiert.

8. Verfahren zur Herstellung der Verbindungen I, dadurch gekennzeichnet, daß man
a) Verbindungen der Formel II

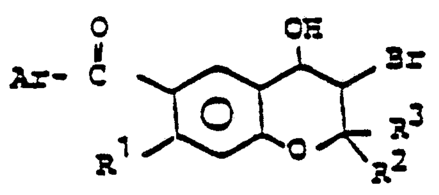

II,

in denen $R^1$ bis $R^3$ und Ar wie oben definiert sind umsetzt mit Lactamen der Formel III

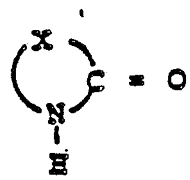

III,

b) Verbindungen der Formel IV

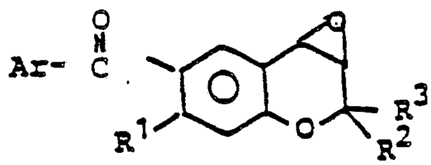

IV,

in denen $R^1$ bis $R^3$ und Ar wie oben definiert sind, umsetzt mit den Lactamen der Formel III

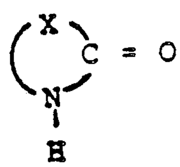

c) Verbindungen der Formel V

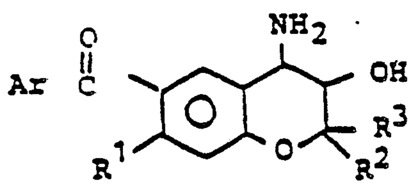

V,

in denen $R^1$ bis $R^3$ und Ar wie oben definiert sind, acyliert zu den Verbindungen VI

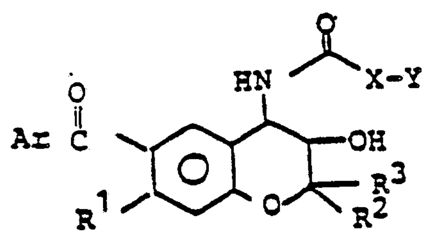

VI,

in denen Y eine Fluchtgruppe, wie etwa Chlor oder Brom, ist und $R^1$ bis $R^3$ und Ar wie oben definiert sind, und diese zu den Verbindungen I cyclisiert

d) Verbindungen der Formel VII

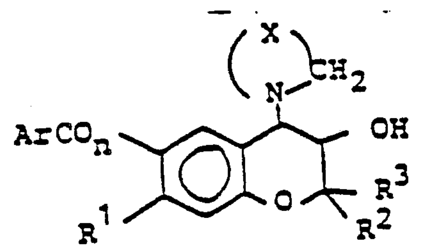

VII,

in denen $r^1$ bis $R^3$ und Ar wie oben definiert sind, zu den Verbindungen I oxidiert.

9. Verwendung einer Verbindung I nach Anspruch 1 zur Herstellung eines Arzneimittels zur Behandlung der Hypertonie, der Angina pectoris, der herzinsuffizienz, von peripheren Durchblutungsstörungen, von Darmmotilitätsstörungen, vorzeitiger Wehentätigkeit, Obstruktionen der Atemwege, der ableitenden Harn- oder Gallenwege oder von Krampfzuständen.

10. Arzneimittel zur Behandlung der Hypertonie, der Angina pectoris, der Herzinsuffizienz, von peripheren Durchblutungsstörungen, von Darmmotilitätsstörungen, vorzeitiger Wehentätigkeit, Obstruktionen der Atemwege, der ableitenden Harn- oder Gallenwege oder von Krampfzuständen, gekennzeichnet durch einen wirksamen Gehalt einer Verbindung I nach Anspruch 1.

Patentansprüche für folgende Vertragsstaaten : ES, GR

1. Verfahren zur Herstellung einer Verbindung I

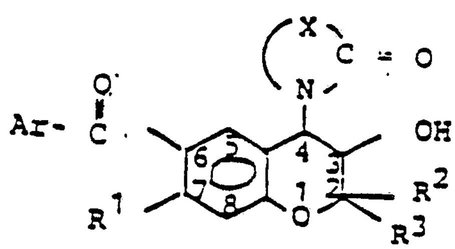

I

in welcher
$R^1$ für H, OH, $(C_1\text{-}C_2)$-Alkoxy, $(C_1\text{-}C_2)$-Alkyl oder $NR^4R^5$ steht, wobei $R^4$und $R^5$ gleich oder verschieden sind und für H, $(C_1\text{-}C_2)$Alkyl oder $(C_1\text{-}C_3)$-Alkylcarbonyl stehen,
$R^2$,$R^3$ gleich oder verschieden sind und für H oder Alkyl mit 1-4 C-Atomen stehen,
$R^2$,$R^3$ zusammen eine Kette $(CH_2)_n$ bilden mit n = 3,4,5 oder 6
Ar für ein aromatisches oder heteroaromatisches System steht, das unsubstituiert oder durch 1 bis 3 gleiche oder verschiedene Reste $(C_1\text{-}C_2)$-Alkyl, $(C_1\text{-}C_2)$-Alkoxy, Halogen, Trifluormethyl, CN, $NO_2$, CO-$(C_1\text{-}C_2)$-Alkyl $SO_m$-$(C_1\text{-}C_2)$-Alkyl mit m = 1 oder 2 substituiert ist
X für eine Kette $(CH_2)_r$ steht, die durch ein Heteroatom 0, S oder $NR^6$ unterbrochen sein kann, wobei $R^6$ H oder $(C_1\text{-}C_4)$-Alkyl bedeutet und r für die Zahlen 2, 3, 4 oder 5 steht
oder
X für eine Kette $(CH)_{n'}$ steht mit $n'$ = 4 oder 6
oder
X für eine Kette $-CH_2-(CH=CH)_{n''}-$ steht mit $n''$ = 1 oder 2,
X unsubstituiert oder substituiert ist durch einen Substituenten A mit A in der Bedeutung $(C_1\text{-}C_2)$-Alkyl, $(C_1\text{-}C_2)$-Alkoxy, COOH, $NO_2$, $NH_2$, Halogen
dadurch gekennzeichnet, daß man

a) Verbindungen der Formel II

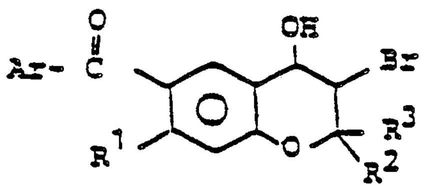

II,

in denen $R^1$ bis $R^3$ und Ar wie oben definiert sind umsetzt mit Lactamen der Formel III

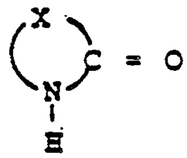

III,

b) Verbindungen der Formel IV

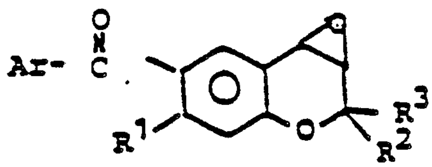

IV,

in denen $R^1$ bis $R^3$ und Ar wie oben definiert sind, umsetzt mit den Lactamen der Formel III

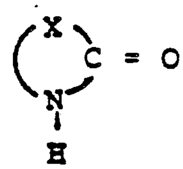

c) Verbindungen der Formel V

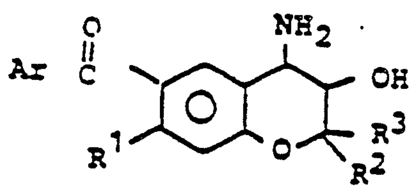

V,

in denen $R^1$ bis $R^3$ und Ar wie oben definiert sind,
acyliert zu den Verbindungen VI

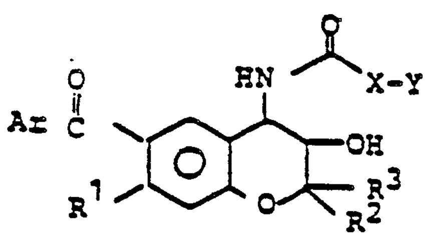

VI,

in denen Y eine Fluchtgruppe, wie etwa Chlor oder Brom, ist und $R^1$ bis $R^3$ und Ar wie oben definiert sind,

und diese zu den Verbindungen I cyclisiert

d) Verbindungen der Formel VII

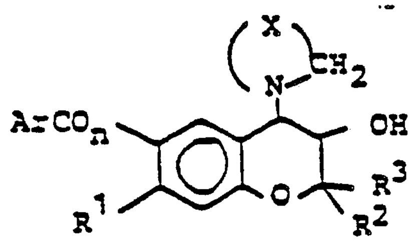

VII,

in denen $R^1$ bis $R^3$ und Ar wie oben definiert sind, zu den Verbindungen I oxidiert.

2. Verfahren nach Anspruch 1, dadurch gekennzeichnet, daß mindestens einer der Substituenten oder Indices folgende Bedeutung hat:

$R^1$,$R^2$,$R^3$ und Ar wie in Anspruch 1 definiert

X $(CH_2)_r$ mit r = 2,3,4 oder 5, welche Kette unsubstituiert oder wie in Anspruch 1 definiert substituiert ist.

3. Verfahren nach Anspruch 1, dadurch gekennzeichnet, daß mindestens einer der Substituenten oder Indices folgende Bedeutung hat:

$R^1$,$R^2$,$R^3$ und Ar wie in Anspruch 1 definiert

X $(CH)_{n'}$ mit $n'$ gleich 4.

4. Verfahren nach Anspruch 1, dadurch gekennzeichnet, daß mindestens einer der Substituenten oder Indices folgende Bedeutung hat:

$R^1$,$R^2$,$R^3$,X wie in Anspruch 1 definiert

Ar Phenyl, das unsubstituiert ist oder einfach substituiert durch Halogen, $(C_1$-$C_2)$-Alkyl, $(C_1$-$C_2)$-Alkoxy.

5. Verfahren nach Anspruch 1, dadurch gekennzeichnet, daß mindestens einer der Substituenten oder Indices folgende Bedeutung hat:

$R^1$,$R^2$,$R^3$ wie in Anspruch 1 definiert

X $(CH_2)_r$ mit R = 3 oder 4

Ar Phenyl, unsubstituiert oder einfach durch Halogen, $(C_1$-$C_2)$-Alkyl, $(C_1$-$C_2)$-Alkoxy substituiert.

6. Verfahren nach Anspruch 1, dadurch gekennzeichnet, daß mindestens einer der Substituenten oder Indices folgende Bedeutung hat:

$R^1$ H

$R^2$,$R^3$ Ar und X wie in Anspruch 1 definiert.

7. Verfahren I nach Anspruch 6, dadurch gekennzeichnet, daß mindestens einer der Substituenten oder Indices folgende Bedeutung hat:

$R^1$ H

$R^2$ und $R^3$ $(C_1$-$C_2)$-Alkyl,

X $(CH_2)_r$ mit r = 3 oder 4

Ar Phenyl, unsubstituiert oder einfach durch Halogen, $(C_1$-$C_2)$-Alkyl, $(C_1$-$C_2)$-Alkoxy substituiert.

8. Verwendung einer Verbindung I nach Anspruch 1 zur Herstellung eines Arzneimittels zur Behandlung der Hypertonie, der Angina pectoris, der Herzinsuffizienz, von peripheren Durchblutungsstörungen, von Darmmotilitätsstörungen, vorzeitiger Wehentätigkeit, Obstruktionen der Atemwege, der ableitenden Harn- oder Gallenwege oder von Krampfzuständen.

Europäisches Patentamt

# EUROPÄISCHER RECHERCHENBERICHT

Nummer der Anmeldung

EP 89114616.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP - B1 - 0 120 427 (BEECHAM) * Ansprüche 1,9,10 * -- | 1,9,10 | C 07 D 405/04<br>C 07 D 405/14<br>A 61 K 31/44<br>A 61 K 31/40 |
| A | DE - A1 - 3 726 261 (MERCK) * Ansprüche 1,8 * -- | 1,9 | |
| A | DE - A1 - 2 713 670 (BEECHAM) * Ansprüche 1,28 * -- | 1,10 | |
| D,A | EP - A2 - 0 277 611 (HOECHST) * Ansprüche 1,10,11 * -- | 1,9,10 | |
| D,A | EP - A2 - 0 277 612 (HOECHST) * Ansprüche 1,7-10 * -- | 1,9,10 | |
| A | EP - B1 - 0 107 423 (BEECHAM) * Ansprüche 1,10-12 * -- | 1,9,10 | RECHERCHIERTE SACHGEBIETE (Int. Cl.4) |
| A | EP - B1 - 0 028 449 (BEECHAM) * Ansprüche 1,10,11 * -- | 1,9,10 | C 07 D 405/00 |
| A | EP - B1 - 0 076 075 (BEECHAM) * Ansprüche 1,24,25 * -- | 1,9,10 | |
| A | CHEMICAL ABSTRACTS, Band 105, Nr. 19, 10. November 1986, Columbus, Ohio, USA ASHWOOD, VALERIE A. et al. "Synthesis and antihypertensive activity of 4-(cyclic amido)-2H-1-benzopyrans." Seite 724, Spalte 2, | 1,10 | |

Der vorliegende Recherchenbericht wurde für alle Patentansprüche erstellt.

| Recherchenort | Abschlußdatum der Recherche | Prüfer |
|---|---|---|
| WIEN | 19-10-1989 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

-2-

EP 89114616.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | Zusammenfassung-Nr. 172 232e & J. Med. Chem. 1986, 29(11), 2194-201 ---- | | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 19-10-1989 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82